# EUROPÄISCHE PATENTSCHRIFT

(11) **EP 0 697 661 B1**
(45) Veröffentlichungstag und Bekanntmachung des Hinweises auf die Patenterteilung: **19.11.1997**
(21) Anmeldenummer: 94112233.5
(22) Anmeldetag: 04.08.1994
(51) Int. Cl.: G06F 11/22, A61C 19/00, A61G 15/02, A61B 19/00

(54) **Einrichtung zur technischen Diagnose von Fehlern in einem ärztlichen, insbesondere zahnärztlichen Gerät**
Apparatus for technical diagnosis of errors in a medical system, in particular a dentist's system
Appareil pour le diagnostic technique d'erreurs dans un système médical, spécialement un système de dentiste

(43) Veröffentlichungstag der Anmeldung: 21.02.1996
(73) Patentinhaber: SIEMENS AKTIENGESELLSCHAFT, 80333 München (DE)
(72) Erfinder: Pabst, Josef, Ing. grad., D-68542 Heddesheim (DE); Pech, Günther, Dipl.-Ing., D-64646 Heppenheim (DE)

(56) Entgegenhaltungen:
- EP-A- 0 140 822
- EP-A- 0 304 848
- EP-A- 0 455 852
- US-A- 4 907 230

## Beschreibung

Die Erfindung betrifft eine Einrichtung zur technischen Diagnose von Fehlern in einem ärztlichen, insbesondere zahnärztlichen Gerät gemäß dem Merkmal a) des Patentanspruches 1.

Medizinische, insbesondere zahnmedizinische Geräte umfassen in der Regel eine Vielzahl von Gerätekomponenten, die eine oder mehrere Flachbaugruppen mit elektronischen Schaltkreisen enthalten, welche auf elektrische und/oder elektromechanische Funktionselemente einwirken. Bei einem zahnärztlichen Gerät können solche Einzelkomponenten sein ein Patientenlagerungsstuhl, ein Arzt- und Helferinnenelement, Tragarme zur Halterung und Verstellung der beiden Elemente, eine Wassereinheit mit Einrichtungen zum Spülen und Saugen sowie zur Wasseraufbereitung, eine Fußsteuereinrichtung, mit der sich u.a. die genannten Funktionselemente ansteuern lassen sowie ein Anschlußkasten, indem eine Versorgungseinheit sowie diverse Ventile für die Medien Luft und Wasser untergebracht sind.

Für den Servicefachmann ist es bei der Vielzahl von Möglichkeiten schwierig, auftretende Fehler relativ rasch zu finden und zu beheben.

Wenn z.B. der elektrische Bohrantrieb beim Arztelement nicht oder nicht einwandfrei funktioniert, so kann dafür eine Vielzahl von Fehlermöglichkeiten in Frage kommen. Als nächstliegende Ursache kann der Motor im Handstück selbst defekt sein; es kann jedoch auch die Eingangsspannung im Anschlußkasten oder eine andere, dazwischenliegende Störstelle die Ursache für den Defekt sein. Durch die elektrische Verknüpfung der Gerätekomponenten kann die Ursache für das Nichtfunktionieren des Motors auch in Bauteilen liegen, die nicht direkt mit dem Motor in Verbindung stehen.

Ziel der vorliegenden Erfindung ist es, eine Diagnoseeinrichtung zu schaffen, mit der es möglich ist, Fehler ohne umständliches und zeitaufwendiges Suchen, welches nach bisheriger Art meist mit einem Zerlegen bzw. Teilzerlegen der Gerätekomponenten verbunden war, lokalisieren zu können.

Das gestellte Ziel wird durch die Merkmale b) bis h) des Patentanspruchs 1 gelöst.

Der Service-Rechner kann in vorteilhafter Ausgestaltung der Erfindung integraler Bestandteil der Einrichtung und einer der Geräte-Komponenten zugeordnet sein; vorteilhaft kann es auch sein, den Service-Rechner extern der Geräte-Komponenten anzuordnen.
Figur 1 zeigt einen zahnärztlichen Arbeitsplatz
Figur 2 zeigt eine Prinzipdarstellung der Verknüpfung der Elemente einer Gerätekomponente
Figur 3 zeigt eine schematische Übersicht einer Ausführungs form der Erfindung
Figur 4 zeigt ein Beispiel für die logische Verknüpfung der Testergebnisse.

Ein Ausführungsbeispiel der Erfindung wird nachfolgend anhand der Zeichnungen näher erläutert.

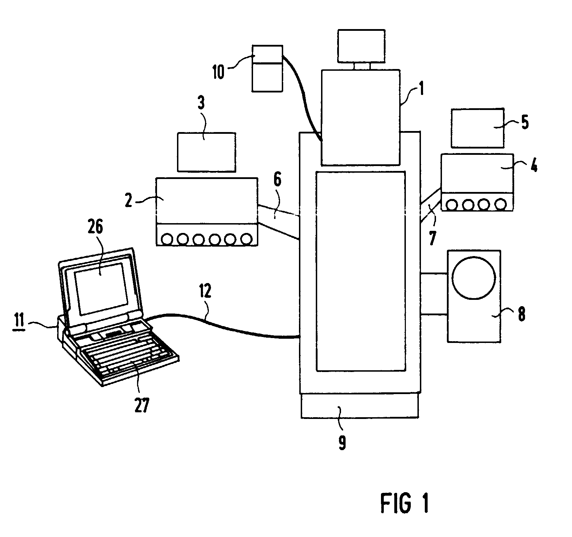

Die Figur 1 zeigt in einer vereinfachten Darstellung in der Draufsicht einen zahnärztlichen Arbeitsplatz, der folgende Geräte-Einzelkomponenten enthält:

Einen Patientenlagerungsstuhl 1, ein Arztelement 2 mit einem Bedien- und Anzeigeteil 3, ein Helferinnenelement 4 mit zugeordnetem Bedien- und Anzeigeteil 5, Tragarme 6 und 7 für die vorgenannten Elemente 2 und 4, eine Wassereinheit 8, einen Anschlußkasten 9 und einen Fußschalter 10.

Mit 11 ist ein Sevice-Rechner bezeichnet, der hier als Laptop ausgebildet ist und, wie nachfolgend noch näher erläutert, über ein Kabel 12 mit den Geräte-Komponenten verbunden ist. Anstelle des Laptop kann auch ein PC oder ein anderer geeigneter Rechner (Notebook, Palmtop) vorgesehen sein.

Zumindest einige der Gerätekomponenten 1 bis 10 enthalten jeweils mindestens eine Flachbaugruppe mit elektronischen Schaltkreisen, die auf elektrische und/oder elektromechanische Funktionselemente einwirken. Elektrische Funktionselemente können beispielsweise Anzeige- und/oder Beleuchtungselemente oder auch Ultraschall- und/oder HF-Chirurgiebausteine sein. Elektromechanische Funktionselemente können Elektromotore und/oder Magnetventile sein. Elektromotore können als Antriebe sowohl in zahnärztlichen Instrumenten angeordnet sein; sie können gleichsam auch als Verstellmotore in den Gerätekomponenten 1, 2 und 4 sowie 6 und 7 angeordnet sein.

Den einzelnen Gerätekomponenten sind analoge und digitale Sensoren zugeordnet, die als Signalgeber wirken und Statusinformationen von den Funktionselementen liefern. Beispiele für solche Statusinformationen sind, Schaltzustände, das Vorhandensein von Spannungen, Temperaturen oder Druck, sowie das Einhalten vorgegebener Sollwerte aufzuzeigen.

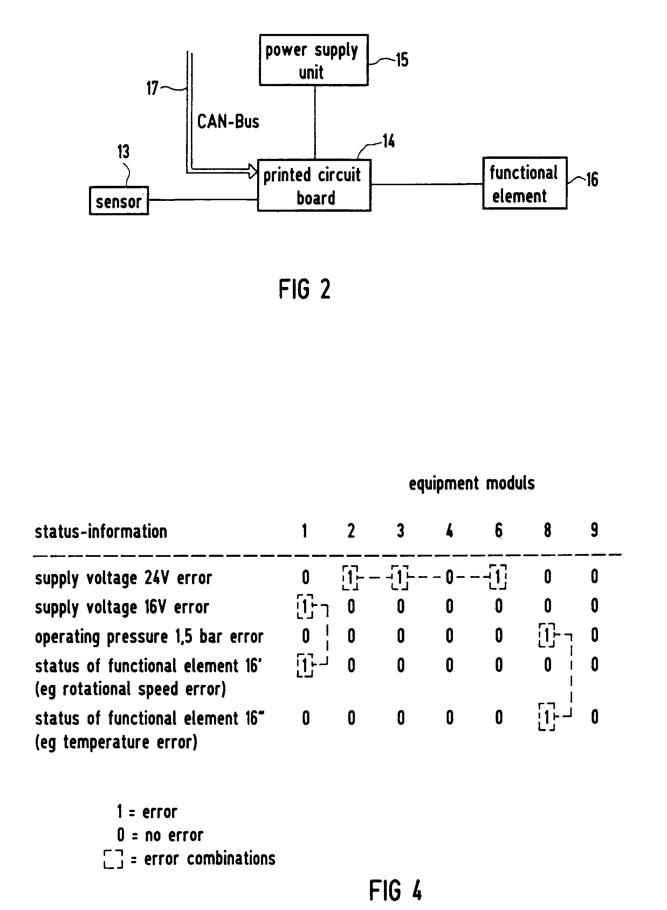

Die Figur 2 zeigt in einer Prinzipdarstellung die Verknüpfung dieser Elemente. Ein Sensor 13 ist mit einer Flachbaugruppe 14 verbunden und liefert an diese ein entsprechendes Statussignal. Die Flachbaugruppe 14, welche von einer Versorgungseinheit 15 mit der notwendigen Spannung versorgt wird, aktiviert das betreffende Funktionselement 16, z.B. ein Steuerventil oder einen Elektroantriebsmotor.

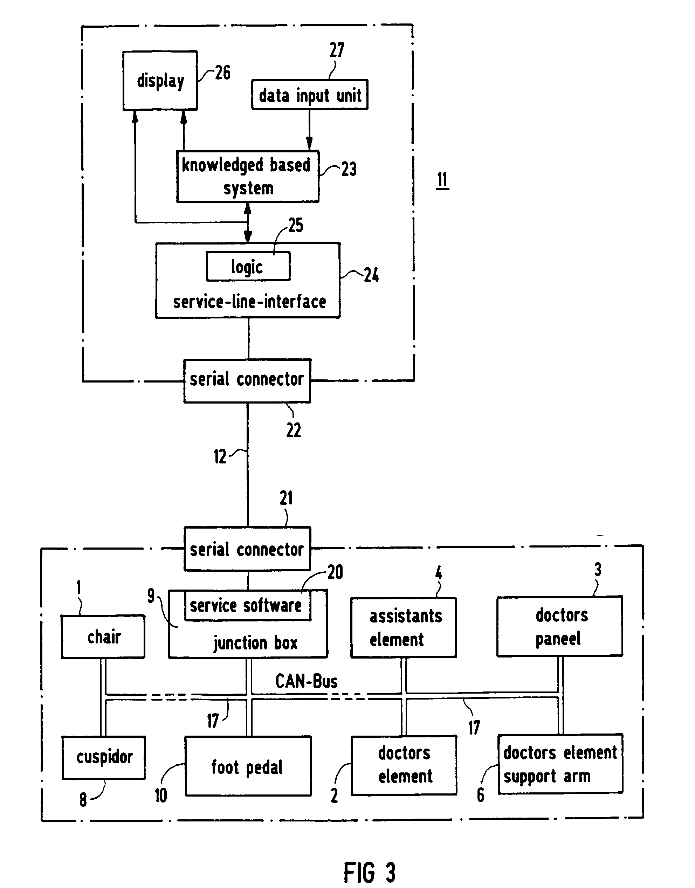

Aus Figur 3 ist ersichtlich, daß sämtliche Flachbaugruppen 14, 14' über einen seriellen Kommunikations-Bus 17 miteinander kommunizieren. Im Ausführungsbeispiel ist angenommen, daß nicht sämtliche Einzelgerätekomponenten 1 bis 10 über den Kommunikations-Bus 17 verbunden sind, sondern nur die Komponenten 1 bis 4, 6, 8 und 9, die Flachbaugruppen aufweisen, welche auf elektrische und/oder elektromechanische Funktionselemente einwirken.

Die Einrichtung enthält eine spezielle Service-Software 20, deren Baustein zweckmäßigerweise im Anschlußkasten 9 untergebracht ist (Fig. 3). Diese Service-Software dient dazu, die für die Diagnose wichtigen Daten für die Übertragung über die serielle Standardschnittstelle zu konvertieren. Über das Verbindungskabel 12 und geeignete Anschlußstecker 21, 22 ist somit eine Kommunikation mit einem beliebigen Rechner, der eine serielle Schnittstelle hat, möglich.

Der Service-Rechner 11 enthält ein erstes Modul 23 mit einem Diagnose-Software-Baustein, der ein kommerzielles Fehlersuchsystem (z.B. Diagnostic Advisor der Firma Emerald Intelligence, USA) enthält. Dieses kommerzielle Fehlersuchsystem ist ein wissensbasiertes System für eine interaktive Fehlersuche. Es arbeitet auf der Basis der geführten interaktiven Diagnose, d.h. gemäß dem von Fachexperten zuvor eingegebenen Wissen sind vom Benutzer Fragen zu beantworten, und zwar ohne Ausnutzung der Geräte-Status-/Fehlerinformationen im On-line-Betrieb.

Mit dem ersten Modul 23 ist ein zweites Modul 24 verbunden, welches einen Verknüpfungslogik-Baustein 25 enthält. Das Modul 24 nimmt die Umsetzung von Servicebefehlen, die in dem Modul 23 von Fachexperten eingegeben werden, in einen entsprechenden Befehl für die Geräte-Komponenten vor und sendet diesen Befehl über die serielle Standardschnittstelle 12 an die zu diagnostizierenden Gerätekomponenten. Die von diesen Gerätekomponenten zurückgesandten Status- und Fehlerdaten werden in die vorerwähnte Verknüpfungslogik 25 eingelesen und durch logische Verknüpfung der Zustands-/Fehlerdaten entsprechend aufbereitet. Vorteilhafterweise erfolgt die Kommunikation der Komponenten über einen CAN-Bus-Befehl (Controlled Area Network).

Die Fehlerdiagnose läuft wie folgt ab:

Die Flachbaugruppen 14, 14' ..., die die einzelnen Gerätekomponenten 1 bis 10 aufweisen, führen zunächst Selbsttest durch, indem sie die Spannungen der auf den Flachbaugruppen vorgesehenen Sollspannungen sowie die Funktionen interner Schaltkreise testen und außerdem die Teilnehmer am Kommunikations-Bus auf deren Vorhandensein und Funktion in bezug auf die Kommunikation überprüfen.

Die Selbsttests auf den Flachbaugruppen liefern Testergebnisse, die über die serielle Schnittstelle 12 als Eingangsgröße dem Service-Rechner 11 zugeführt werden. Die in den Rechner eingegebenen Testergebnisse werden in der Verknüpfungslogik 25 logisch miteinander verknüpft. Die Verknüpfung erfolgt dabei entsprechend einer zweidimensionalen Matrix, die am Beispiel der Figur 4 näher erläutert wird.

Die in den einzelnen Geräte-Komponenten 1 bis 4, sowie 6, 8, und 9 aufgrund der Ergebnisse der Selbsttestfunktion registrierten Fehler werden in die Verknüpfungslogik eingelesen und über logische Gleichungen, die im Sofware-Baustein enthalten sind, verknüpft. Der Aufbau dieser Gleichungen basiert auf dem Gedanken, daß sich ein Fehler bzw. eine fehlerhafte Funktion in einer Geräte-Komponente in einem bestimmten Fehlerbild, d.h. im Auftreten einer bestimmten Kombination von Fehlern äußert.

Im Ausführungsbeispiel nach Fig.4 wird dem Diagnose-System ein Spannungsfehler in der 24-Volt Versorgungsspannung gemeldet, und zwar jeweils von den Komponenten 2,3 und 8 , also von Arztgerät, Arzt-Bedienpaneel und Arzt-Tragarm. Da die drei Komponenten physikalisch direkt miteinander verbunden sind und eine Einheit bilden, ist die Wahrscheinlichkeit sehr hoch, daß nicht eine Leitungsunterbrechung der 24-Volt-Versorgung bei allen drei Komponenten vorliegt, sondern daß die zentrale Sicherung im Versorgungsbereich für die Einheit defekt ist. Die Verknüpfung kann im Ausführungsbeispiel also so sein, daß, wenn bei Komp. 1 ein 24V Fehler und bei Komp. 6 ein 24V Fehler und bei Komp. 3 ein 24V Fehler vorliegt, dann die Diagnose gegeben wird, daß die Zentralsicherung defekt ist.

Die Diagnose kann z.B. verschlüsselt am Display 26 des Rechners 11 aufscheinen.

Der Vorteil der Verknüpfung dieser Testergebnisse besteht darin, daß im aufgezeigten Falle die Spannungsversorgungswege der einzelnen Komponenten nicht der Reihe nach überprüft werden müssen, bevor man letztlich dann feststellt, daß die zentrale Sicherung defekt ist.

Bei einer ausreichenden Lokalisierung des Fehlers wird eine entsprechende Anweisung direkt an den Bildschirm gegeben. Ist der Fehler nicht ausreichend lokalisiert, können Statusinformationen von Funktionselementen der einzelnen Geräte-Komponenten, z.B Magnetventile, Lampen oder Motoren, abgefragt werden. Diese Daten dienen sodann als zusätzliche Informationen für eine erneut zu startende Diagnose.

Ist auch bis jetzt noch keine ausreichende Lokalisierung des Fehlers gegeben, so wird die Diagnose interaktiv innerhalb des Moduls 23 fortgesetzt. Die interaktiv über eine Eingabeeinheit 27 (z.B. Tastatur des Rechners) gegebenen Antworten fließen in die Verknüpfungslogik 25 zurück und werden dort erneut so lange verknüpft, bis eine eindeutige Fehlerdiagnose gegeben ist. Die interaktiv erhaltenen Informationen ergänzen dabei die bereits vorliegenden Daten und gehen in die Verknüpfungsgleichung für das typische Fehlerbild des aufgetretenen Fehlers ein was schließlich im Ergebnis zu einer genaueren Lokalisierung des Fehlers führt.

Neben dem obengenannten Ausführungsbeispiel einer Fehlerdiagnose mit einer Fehlerursache im Bereich der 24 Volt Versorgungsspannung gilt Entsprechendes für die Verknüpfung von Fehlern unterschiedlicher Arten, z.B. fehlerhafte Schaltzustände, Betriebsdrücke, Temperaturen usw., wie dies in Figur 4 angedeutet ist. Danach kann beispielsweise eine Fehlermeldung in der 16-Volt-Betriebsspannung in der Geräte-Komponente 1 mit einer Statusmeldung eines Funktionselementes 16', z.B. mit einem Drehzahlfehler eines Antriebsmotors in der Geräte-Komponente 1, verknüpft werden. Ebenso kann eine Fehlermeldung im Betriebsdruck eines Mediums in der Geräte-Komponente 8 (Wassereinheit) mit einer Temperatur-Fehlermeldung in dieser Geräte-Komponente verknüpft werden.

## Patentansprüche

1. Einrichtung zur technischen Diagnose von Fehlern in einem ärztlichen, insbesondere zahnärztlichen Gerät, **gekennzeichnet** durch folgende Merkmale:
a ) das Gerät enthält mehrere Komponenten (1 bis 10 ), die Flachbaugruppen (14, 14', ...) mit elektronischen Schaltkreisen enthalten, welche auf elektrische und/oder elektromechanische Funktionselemente (16, 16', ...) einwirken,
b ) die Flachbaugruppen (14, 14', ...) kommunizieren über einen seriellen Kommunikations-Bus (17) miteinander,
c ) den Komponenten ( 1 bis 10 ) sind analoge und digitale Signalgeber (13, 13', ...) zugeordnet, deren Signale als Eingangsgröße auf die Flachbaugruppen wirken,
d ) die Flachbaugruppen (14, 14', ...) enthalten Mittel zur Durchführung von Selbsttests, indem sie die auf den Flachbaugruppen vorgesehenen Spannungen sowie die Funktionen interner Schaltkreise testen und die Teilnehmer am Kommunikations-Bus auf deren Vorhandensein und korrekte Funktion in bezug auf eine Kommunikation überprüfen,
e) die Selbsttests liefern Testergebnisse, die als Eingangsgrößen einem Service-Rechner (11) zugeführt werden,
f ) zur Übertragung der Daten auf den Service-Rechner (11) sind Mittel (20) vorgesehen, welche die Daten für eine Übertragung über eine Standard-Schnittstelle konvertieren,
g) der Service-Rechner (11) enthält ein erstes Modul (23), welches eine interaktive auf Benutzereingaben basierende Fehlerdiagnose off-line ermöglicht, und ein zweites Modul (24), welches eine Verbindung zwischen dem ersten Modul (23) und den Geräte-Komponenten ( 1 bis 10) herstellt, um eine on-line Diagnose mit dem ersten Modul (23) zu ermöglichen,
h) das zweite Modul (24) enhält eine Verknüpfungslogik (25), die die Testergebnisse logisch miteinander verknüpft und zu einem Diagnoseergebnis führt, welches bei ausreichender Lokalisierung auf einem Display (26) aufscheint und welches, wenn die angezeigte Diagnose noch nicht genau genug ist, an das erste Modul (23) zurückgegeben wird und dort interaktiv weiterbearbeitet wird, wobei interaktiv gegebene Antworten in die Verknüpfungslogik (25) zurückfließen und dort erneut solange verknüpft werden, bis sich eine eindeutige Fehlerdiagnose ergibt.

2. Einrichtung nach Anspruch 1 , **dadurch gekennzeichnet,** daß der Service-Rechner (11) integraler Bestandteil einer der Geräte-Komponenten (1 bis 10), vorteilhafterweise des dem Behandler zugeordneten Bedien- und Anzeigepaneels (3), ist.

3. Einrichtung nach Anspruch 1, **dadurch gekennzeichnet,** daß der Service-Rechner (11) ein mobiles Datenverarbeitungsgerät ist.

4. Einrichtung nach Anspruch 3, **dadurch gekennzeichnet**, daß der Rechner (11) über ein Steckverbindungskabel (12) an die Standard-Schnittstelle einer der Geräte-Komponenten (1 bis 10) angeschlossen ist.

## Claims

1. Device for the technical diagnosis of errors in a medical, in particular a dental, apparatus,
characterised by the following features:
a) the apparatus contains a plurality of components (1 to 10) which contain printed-circuit boards (14, 14', ...) with electronic circuits which act upon electrical and/or electromechanical functional elements (16, 16', ...),
b) the printed-circuit boards (14, 14', ...) communicate with each other by way of a serial communications bus (17),
c) associated with the components (1 to 10) there are analog and digital signal transmitters (13, 13', ...), the signals of which act as an input variable on the printed-circuit boards,
d) the printed-circuit boards (14, 14', ...) contain means for carrying out self-tests, as they test the voltages on the printed-circuit boards and also the functions of internal circuits and check the parties on the communications bus to check that they are present and functioning correctly with regard to communication,
e) the self-tests supply test results which are fed as input variables to a service computer (11),
f) for the transmission of the data to the service computer (11) means (20) are provided that convert the data for transmission by way of a standard interface,
g) the service computer (11) contains a first module (23), which renders possible interactive, user input-based error diagnosis off-line, and a second module (24) which establishes a connection between the first module (23) and the apparatus-components (1 to 10) in order to render possible on-line diagnosis with the first module (23),
h) the second module (24) contains a logic element (25) which combines the test results together in a logic operation and produces a diagnosis result which, given sufficient localization, appears on a display (26) and which, if the diagnosis which is displayed is not yet sufficiently accurate, is returned to the first module (23) and there processed further in an interactive manner, with answers given in an interactive manner flowing back into the logic element (25) and there undergoing a logic operation once again for so long until an unambiguous error diagnosis results.

2. Device according to claim 1, characterised in that the service computer (11) is an integral part of one of the apparatus-components (1 to 10), advantageously of the operator and display panel (3) which is associated with the attendant.

3. Device according to claim 1, characterised in that the service computer (11) is a mobile data-processing unit.

4. Device according to claim 3, characterised in that the computer (11) is connected to the standard interface of one of the apparatus-components (1 to 10) by way of a plug-in connection cable (12).

## Revendications

1. Dispositif de diagnostic technique de défauts dans un appareil médical, notamment dans un appareil de médecine dentaire, caractérisé par les dispositions suivantes:
a) l'appareil comprend plusieurs composantes (1 à 10), qui contiennent des modules (14, 14',...) plats comportants des circuits de commande électroniques, qui agissent sur des éléments (16, 16',...) fonctionnels électriques et/ou électromécaniques,
b) les modules (14, 14',...) plats communiquent entre eux par l'intermédiaire d'un bus (17) de communication sériel,
c) il est associé aux composantes (1 à 10) des générateurs (13, 13',...) de signaux analogiques et numériques dont les signaux agissent comme grandeur d'entrée sur les modules plats,
d) les modules (14, 14',...) plats contiennent des moyen d'effectuer des autotests, en testant les tensions prévues sur les modules plats ainsi que les fonctionnements de circuits de commande internes et en vérifiant la présence et le fonctionnement correct en ce qui concerne une communication des participants au bus de communication,
e) les autotests donnent des résultats de test qui sont envoyés à un ordinateur (11) de maintenance comme grandeurs d'entrée,
f) pour transmettre les données à l'ordinateur (11) de maintenance, il est prévu des moyens qui convertissent les données pour une transmission par l'intermédiaire d'une interface normalisée,
g) l'ordinateur (11) de maintenance contient un premier module (23), qui permet de manière autonome un diagnostic de défaut interactif, fondé sur des entrées d'utilisateur, et un deuxième module (24), qui produit une liaison entre le premier module (23) et les composantes (1 à 10) de l'appareil afin de permettre un diagnostic interconnecté par le premier module (23),
h) le deuxième module (24) contient une logique (25) de combinaison, qui combine logiquement entre eux les résultats de test et donne un résultat de diagnostic qui, lorsque la localisation est suffisante, apparaît sur un écran (26) et qui, si le diagnostic indiqué n'est pas encore assez précis, est renvoyé au premier module (23) et y est traité de manière interactive, des réponses données de manière interactive revenant à la logique (25) de combinaison et y étant à nouveau combinées jusqu'à ce que l'on obtienne un diagnostic de défaut clair.

2. Dispositif suivant la revendication 1, caractérisé en ce que l'ordinateur (11) de maintenance fait partie intégrante de l'une des composantes (1 à 10) de l'appareil, avantageusement du tableau (3) de commande et d'indication associé à la personne soignante.

3. Dispositif suivant la revendication 1, caractérisé en ce que l'ordinateur (11) de maintenance est un appareil de traitement de données mobile.

4. Dispositif suivant la revendication 3, caractérisé en ce que l'ordinateur (11) est raccordé à l'interface normalisée de l'une des composantes (1 à 10) de l'appareil par l'intermédiaire d'un câble (12) de liaison enfichable.
